# EUROPÄISCHE PATENTSCHRIFT

(11) **EP 0 832 602 B1**
(45) Veröffentlichungstag und Bekanntmachung des Hinweises auf die Patenterteilung: **17.03.2004**
(21) Anmeldenummer: 96810640.1
(22) Anmeldetag: 27.09.1996
(51) Int. Cl.: A61B 5/042, A61M 25/01, A61M 29/02

(54) **Einrichtung zur Durchführung von diagnostischen und/oder therapeutischen Herzeingriffen mit Katheter**
Device for carrying out diagnostic and/or therapeutic heart procedures with a catheter
Dispositif pour exécuter des interventions diagnostiques et/ou thérapeutiques du coeur à l'aide de cathéter

(43) Veröffentlichungstag der Anmeldung: 01.04.1998
(73) Patentinhaber: Sulzer Osypka GmbH, 79639 Grenzach-Wyhlen (DE)
(72) Erfinder: Osypka, Peter, Dr., 79639 Grenzach-Wyhlen (DE)
(74) Vertreter: Maucher, Wolfgang, Dipl.-Ing.

(56) Entgegenhaltungen:
- EP-A- 0 659 388
- EP-A- 0 692 221
- EP-A- 0 711 573
- WO-A-94/02077
- US-A- 5 140 987
- US-A- 5 417 669
- US-A- 5 487 385

## Beschreibung

Die Erfindung betrifft eine Einrichtung zur Durchführung von diagnostischen und/oder therapeutischen Herzeingriffen mit Katheter gemäss Oberbegriff von Anspruch 1.

Eine vergleichbare Einrichtung ist aus der EP-A 0 692 221 bekannt, nämlich ein Katheter mit einem Führungsschlauch und einer Mehrfachelektrode. Dieser Katheter hat an seinem distalen Ende eine Stützschlinge aus biegsamem oder flexiblem Werkstoff, die aus dem Führungsschlauch ausgeschoben und in ihrem Umfang vergrössert oder verkleinert werden kann. Die Stützschlinge dient bei einer besonderen Ausführungsform (siehe Fig. 6 der EP-A 0 692 221) als Halter für einen weiteren, ebenfalls aus demselben Führungsschlauch ausschiebbaren Führungsdraht. Der Führungsdraht ist dabei mit der Stützschlinge über eine auf der Stützschlinge beweglichen Öse verbunden. Durch Drehen und Schieben des Führungsdrahts kann dieser, stabilisiert durch die Öse an der Stützschlinge, in einem Teilraum des Herzes den Wandkonturen folgend an die Innenwand angelegt werden. Als Mehrfachelektrode ist eine Mehrfachwendel vorgesehen, in deren inneren Kanal sich der Führungsdraht erstreckt. Durch eine gesteuerte Lageänderung des Führungsdrahts und Verschieben der Mehrfachelektrode entlang des Führungsdrahts lässt sich die Herzinnenwand abtasten. Mit einer derartigen Einrichtung wird die Wand Streifen für Streifen behandelt, wobei jedem Streifen jeweils eine vorgesehene Positionierung des Führungsdrahts zugeordnet ist. Aufgrund der Pumpbewegung des Herzes kann es Probleme bezüglich einer stabilen Positionierung des Führungsdrahts und der Stützschlinge von außen durch bzw. über den gemeinsamen Führungsschlauch geben.

Es ist Aufgabe der Erfindung, eine gattungsgemäße Einrichtung zu schaffen, mit der die genannte streifenartige Behandlung bei jeweils stabil positioniertem Führungsdraht - auch bei Vorliegen von besonders glatten Stellen der Herzinnenwand - durchführbar ist.

Diese Aufgabe wird durch eine Einrichtung mit den Merkmalen des Anspruchs 1 gelöst. Diese Einrichtung zur Durchführung von diagnostischen und/oder therapeutischen Herzeingriffen umfasst einen Katheter, der einen geführten Katheter, einen den geführten Katheter umschliessenden Führungskatheter und ein in einem Lumen des geführten Katheters sich erstreckenden Führungsdraht. Es sind Mittel vorgesehen, die eine gesteuerte Lageänderung des Führungsdrahts in einem Teilraum des Herzes erlauben. Der Katheter ist ein erster Katheter, dessen Führungsdraht mit dem distalen Ende eines zweiten Katheters in Verbindung steht bzw. mit diesem verbindbar ist. Der zweite Katheter ist im Herz derart positionierbar, dass sein distales Ende eine von der Lageänderung des Führungsdrahts unabhängige stationäre Lage einnimmt.

Durch die erfindungsgemäße Verwendung eines Führungsdrahts, der zwischen den distalen Enden zweier Katheter angeordnet sowie zwischen diesen Enden bewegbar ist, erhält man eine gute Stabilität des Führungsdrahtes. Denn die distalen Enden der Katheter lassen sich im Herz oder am Eingang des Herzes so positionieren, dass sie trotz der Herzbewegung stationäre Lagen einnehmen. Diese stationären Lagen stellen für den Führungsdraht fest vorgegebene Randbedingungen dar, die dem beweglichen Draht die nötige Stabilität vermitteln. Der Führungsdraht lässt sich in der erfindungsgemäßen Einrichtung von beiden Seiten her durch Drehen und Schieben manipulieren.

Die abhängigen Ansprüche 2 bis 8 betreffen diverse Ausführungsformen der erfindungsgemässen Einrichtung.

Nachfolgend wird die Erfindung anhand der Zeichnung näher beschrieben. Es zeigen:
- Fig. 1: einen Katheter, der einen Teil der erfindungsgemäßen Einrichtung bildet,
- Fig. 2: ein Detail des in Fig. 1 abgebildeten Katheters,
- Fig. 3: die in das Herz eingeführten Teile einer ersten Ausführungsform der erfindungsgemässen Einrichtung,
- Fig. 4: eine der Fig. 3 entsprechenden Darstellung für eine zweite Ausführungsform und
- Fig. 5: die Darstellung einer dritten Ausführungsform.

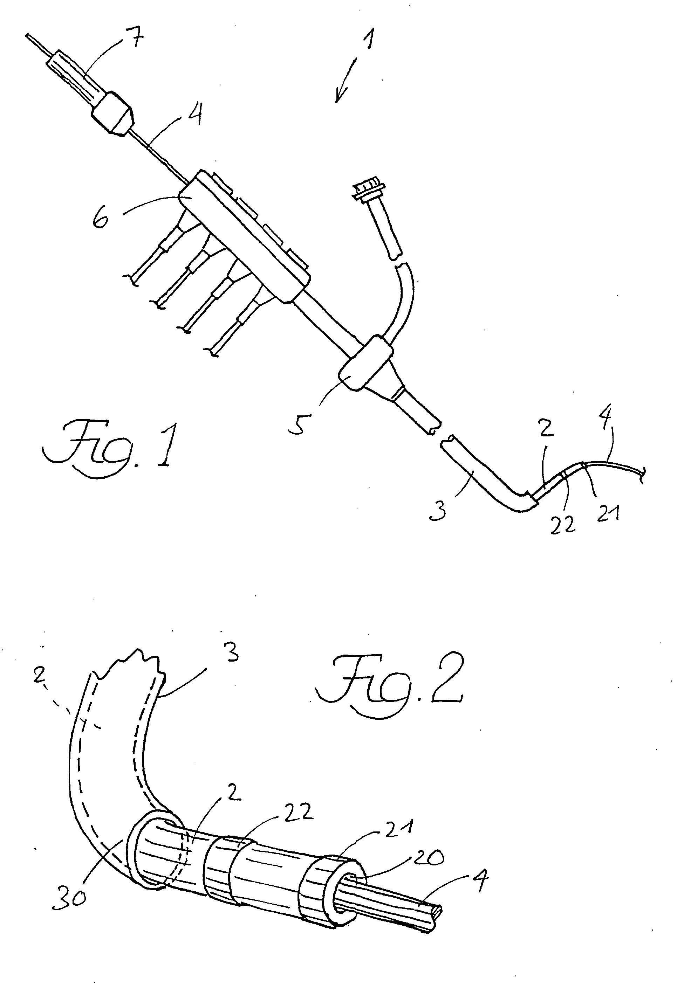

Der Katheter 1 der Fig. 1 umfasst folgende Komponenten: einen geführten Katheter 2 - hier als Elektrokatheter mit Polen 21 und 22 ausgeführt; einen Führungskatheter 3 mit einem distalen Ende 30; einen Führungsdraht 4; eine Schleuse; einen Elektrodenanschluss; ferner einen Griff für das proximale Ende des Führungsdrahts 4.

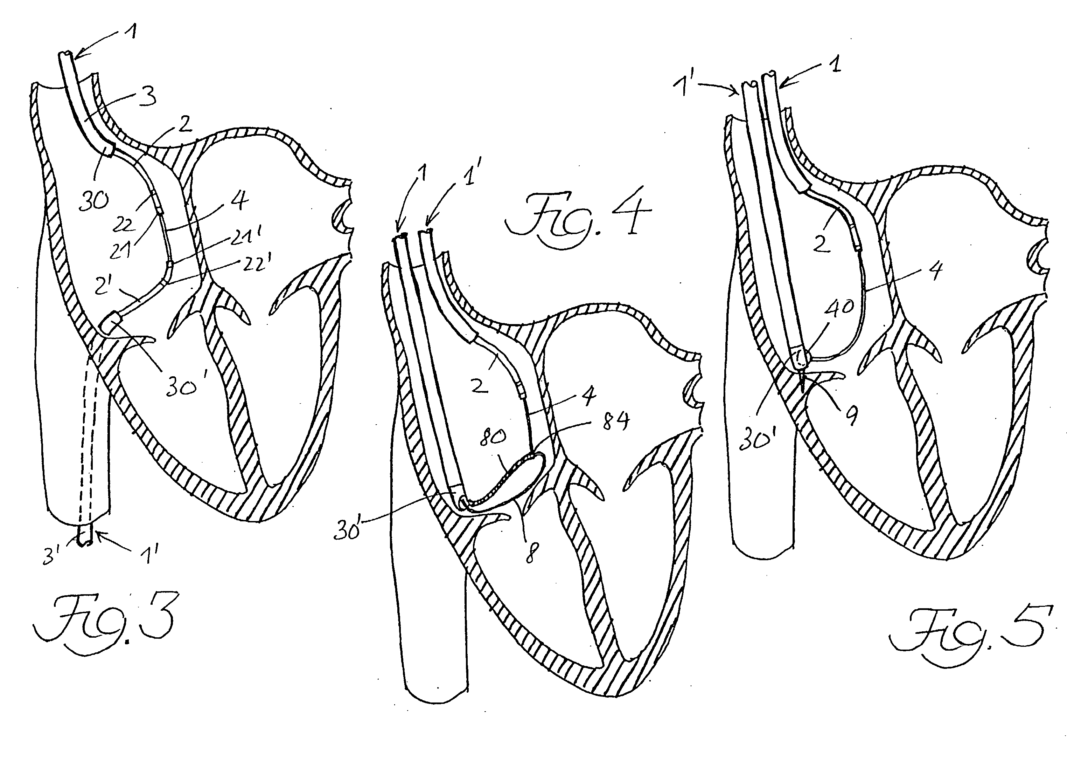

Die perspektivische Ansicht der Fig.2 zeigt das distale Ende 30 des in Fig.1 dargestellten Führungskatheters 3. An dem distalen Ende des geführten Katheters 2 sieht man hier zusätzlich einen inneren Kanal oder Lumen 20, in dem sich der Führungsdraht 4 erstreckt.

Der Führungsdraht 4 wird mit Vorteil der Anatomie des Herzes entsprechend vorgeformt. Für diese Vorformung ist der Werkstoff Nitinol besonders geeignet. Statt Nitinol kommen auch andere biegsame oder flexible Werkstoffe aus metallischen Legierungen oder auch Polymeren in Frage.

Der zweite Katheter 1' der in Fig.3 dargestellten Ausführungsform der Erfindung umfasst einen zweiten geführten Katheter 2'; und die beiden Katheter 1 und 1' weisen einen gemeinsamen Führungsdraht 4 auf.

Der zweite Katheter 1' ist unabhängig von dem ersten Katheter 1 in das Herz einführbar. Der erste Katheter 1 tritt in das Herz über ein erstes Blutgefäss ein, nämlich über die Vena subclavia; der zweite Katheter 1' über ein zweites Blutgefäss, nämlich die Vena femoralis. Beim Einschieben des zweiten Katheters 1' in die Vena femoralis - nicht dargestellt - enthält dieser ein Einfangorgan, beispielsweise ein Fangkatheter mit einer Schlinge, mit dem eine gebogene Spitze des Führungsdrahts 4 des ersten Katheters 1 ergriffen werden kann. Mit dem Einfangorgan wird der Führungsdraht 4 in den zweiten Katheter 1' eingezogen. Der Katheter 1 mit der Schleuse 5 (siehe Fig.1) wie auch der Katheter 1' können mit der bekannten Seldinger-Technik in den Körper des Patienten eingeführt werden.

Kunststoffgriffe 7 (siehe Fig.1), die nach dem Einführen an die beiden Enden des Führungsdrahts 4 angebracht werden können, erleichtern das Manipulieren (Drehen, Stossen, Ziehen) des Drahts 4.

Die geführten Katheter 2 und 2' sind als Elektrodenkatheter ausgebildet. Mit deren Polen 21, 22, 21' und 22' sind Herzpotentiale abtastbar (sogenanntes "mapping"); und/oder es ist mit ihnen Hochfrequenzstrom zur Ablation von Gewebe abgebbar. Es sind insbesondere auch Temperaturfühler bei den zur Ablation dienenden Polen angeordnet (nicht dargestellt), die für eine Regelung des Hochfrequenzstroms zur Vermeidung eines Überhitzens des zu behandelnden Gewebes vorgesehen sind.

Die distalen Enden 30, 30' der beiden Führungskatheter 3, 3' sind unmittelbar an den Eingängen zum rechten Vorhof des Herzes positioniert. Bei diesen Positionierungen ergibt sich für den gemeinsamen Führungsdraht 4 eine stabile, von der Herzbewegung nicht gestörte Lage.

Bei den Ausführungsformen der Figuren 4 und 5 sind die Zuführungen der Katheter in das Herz so vorgesehen, dass sie über das gleiche Blutgefäss, nämlich die Vena subclavia, in das Herz eintreten.

Im Beispiel der Fig.4 enthält das distale Ende 30' des zweiten Katheters 1' eine biegsame Stützschlinge 8, die zur Fixierung des Katheters 1' im Herzen vorgesehen ist; diese ist ausschiebbar ausgebildet. Die Schlinge 8 dient einerseits zur Fixierung des Katheters 1' an einer beliebigen Stelle des Herzes. Andererseits wird auf der Schlinge das distale Ende des Führungsdrahts 4 positioniert, und zwar mit einer Metallwendel 80, die über die Schlinge 8 geschoben ist und an deren Ende 84 der Führungsdraht 4 befestigt ist (vgl. Fig.4 in der EP-A 0 692 221). Durch Zug und Schub der Wendel 80 kann der Führungsdraht 4 innerhalb des Herzes über praktisch den ganzen Umfang von 360° positioniert werden. Durch die Führung über den ersten Katheter 1 wird der Draht 4 gegen die Herzwand gedrückt. Über den Führungsdraht 4 wird - siehe Ausführungsbeispiel (Fig.3) - die eigentliche Mapping- und Ablationselektrode 2 geschoben.

Bei dem Ausführungsbeispiel der Fig.5 enthält das distale Ende 30' des zweiten Katheters 1' eine Spitze 9, die zur Fixierung des Katheters 1' im Herzen vorgesehen ist; sie ist ausfahrbar ausgebildet. Der Führungsdraht 4 des ersten Katheters 1 ist über ein Gelenk 40 mit dem distalen Ende 30' des zweiten Katheters 1' verbunden.

Bei den in den Figuren 3 bis 5 gezeigten Ausführungsformen der erfindungsgemässen Einrichtung sind die geführten Katheter 2 jeweils als Mapping- und Ablationselektrode ausgebildet. Es ist aber zum Beispiel auch möglich, dass die geführten Katheter 2 lichtleitende Fasern enthalten. Mit solchen Fasern lässt sich eine visuelle Diagnose oder eine Kontrolle von Behandlungsschritten durchführen; und/oder es kann Laserstrahlungsenergie zur Behandlung von Gewebe in das Herz eingetragen werden.

## Patentansprüche

1. Einrichtung zur Durchführung von diagnostischen und/oder therapeutischen Herzeingriffen mit einem ersten Katheter (1) und einem zweiten Katheter (1'), der erste Katheter (1) umfasst einen geführten Katheter (2), einen den geführten Katheter (2) umschließenden Führungskatheter (3) und ein in den Lumen (20) des geführten Katheters (2) sich erstreckenden Führungsdraht (4), wobei Mittel (7) vorgesehen sind, die eine gesteuerte Lageänderung des Führungsdrahtes (4) in einen Teilraum des Herzens erlauben und bei der der Katheter ein erster Katheter (1) ist, dessen Führungsdraht mit dem distalen Ende (30') dess zweiten Katheters (1') in Verbindung steht bzw. mit diesem verbindbar ist, wobei der zweite Katheter (1') im Herz derart positionierbar ist, dass sein distales Ende eine von der Lageänderung des Führungsdrahtes unabhängige stationäre Lage einnimmt, wobei der zweite Katheter (1') unabhängig von dem ersten Katheter (1) in das Herz einführbar ist und der zweite Katheter (1') einen zweiten geführten Katheter (2') umfasst, wobei der zweite Katheter (1') ein Einfangorgan umfasst, mit dem der Führungsdraht (4) des ersten Katheters (1) ergreifbar oder in den zweiten Katheter einziehbar ist. So dass der erste (1') und der zweite Katheter (2') einen gemeinsamen Führungsdraht (4) aufweisen.

2. Einrichtung nach Anspruch 1, **dadurch gekennzeichnet, dass** für den gemeinsamen Führungsdraht (4) Griffe (7) vorgesehen sind, die nach dem Einziehen des Führungsdrahtes an dessen beiden Enden anbringbar sind und die das Manipulieren zur gesteuerten Lageänderung des Drahts erleichtern.

3. Einrichtung nach Anspruch 1 oder 2, **dadurch gekennzeichnet, dass** die Zuführungen der Katheter (1, 1') in das Herz so vorgesehen sind, dass der erste Katheter (1) über ein ersten Blutgefäss, insbesondere die Vena subclavia, und der zweite Katheter über ein zweites Blutgefäss, insbesondere die Vena femoralis, in das Herz eintreten.

4. Einrichtung nach Anspruch 1 oder 2, **dadurch gekennzeichnet, dass** die Zuführungen der Katheter (1, 1') in das Herz so vorgesehen sind, dass sie über das gleiche Blutgefäss, insbesondere die Vena subclavia, in das Herz eintreten.

5. Einrichtung nach einem der Ansprüche 1 bis 4, **dadurch gekennzeichnet, dass** das distale Ende (30') des zweiten Katheters (1') eine biegsame Stützschlinge (8) enthält, die zur Fixierung des Katheters im Herzen vorgesehen ist und die ausschiebbar ausgebildet ist.

6. Einrichtung nach einem der Ansprüche 1 bis 4, **dadurch gekennzeichnet, dass** das distale Ende (30') des zweiten Katheters (1') eine Spitze (9) enthält, die zur Fixierung des Katheters im Herzen vorgesehen ist und die ausfahrbar ausgebildet ist.

7. Einrichtung nach einem der Ansprüche 1 bis 6, **dadurch gekennzeichnet, dass** der oder die geführten Katheter (2, 2') als Elektrodenkatheter ausgebildet sind, mit deren Polen (21, 22, 21', 22') Herzpotentiale abtastbar sind und/oder Hochfrequenzstrom zur Ablation von Gewebe abgebbar ist, wobei insbesondere Temperaturfühler bei den Polen für die Ablation angeordnet sind, die für eine Regelung des Hochfrequenzstroms zur Vermeidung eines Überhitzens des zu behandelnden Gewebes vorgesehen sind.

8. Einrichtung nach einem der Ansprüche 1 bis 7, **dadurch gekennzeichnet, dass** der oder die geführten Katheter (2, 2') lichtleitenden Fasern enthalten, mit denen eine visuelle Diagnose oder eine Kontrolle von Behandlungsschritten möglich sind und/oder mit denen Lichtenergie, insbesondere Laserstrahlung, zur Behandlung von Gewebe in das Herz eintragbar ist.

## Claims

1. Device for carrying out diagnostic and/or therapeutic heart interventions, having a first catheter (1) and a second catheter (1'), the first catheter (1) comprises a guided catheter (2), a guide catheter (3) which surrounds the guided catheter (2) and a guide wire (4) which extends into the lumen (20) of the guided catheter (2), means (7) being provided which allow a controlled change of position of the guide wire (4) into a compartment of the heart and wherein the catheter is a first catheter (1) the guide wire of which is connected or connectable to the distal end (30') of the second catheter (1'), the second catheter (1') being adapted to be positioned in the heart such that its distal end assumes a stationary position independent of the change of position of the guide wire, the second catheter (1') being insertable into the heart independently of the first catheter (1) and the second catheter (1') comprising a second guided catheter (2'), the second catheter (1') comprising a capturing member by which the guide wire (4) of the first catheter (1) can be grabbed or pulled into the second catheter, so that the first catheter (1') and the second catheter (2') have a common guide wire (4).

2. Device according to claim 1, **characterised in that** grips (7) are provided for the common guide wire (4), which can be attached to both ends of the guide wire after it has been pulled in and make manipulation easier for a controlled change of position of the wire.

3. Device according to claim 1 or 2, **characterised in that** the leads for the catheters (1, 1') into the heart are arranged such that the first catheter (1) enters the heart through a first blood vessel, particularly the subclavian vein, and the second catheter enters the heart through a second blood vessel, particularly the femoral vein.

4. Device according to claim 1 or 2, **characterised in that** the leads for the catheters (1, 1') into the heart are arranged such that they enter the heart through the same blood vessel, particularly the subclavian vein.

5. Device according to one of claims 1 to 4, **characterised in that** the distal end (30') of the second catheter (1') contains a flexible support loop (8) which is provided for securing the catheter in the heart and is arranged to be capable of being pushed out.

6. Device according to one of claims 1 to 4, **characterised in that** the distal end (30') of the second catheter (1') contains a tip (9) which is provided for fixing the catheter in the heart and is arranged to be extendable.

7. Device according to one of claims 1 to 6, **characterised in that** the guided catheter or catheters (2, 2') is or are constructed as electrode catheters the poles (21, 22, 21', 22') of which can be used to scan heart potentials and/or to emit high frequency current for the ablation of tissue, while temperature sensors, in particular, are disposed at the poles for the ablation, said sensors being provided for regulating the high frequency current in order to avoid overheating the tissue which is to be treated.

8. Device according to one of claims 1 to 7, **characterised in that** the guided catheter or catheters (2, 2') contain light-conducting fibres by means of which visual diagnosis can be carried out or any treatment steps can be monitored and/or by means of which light energy, especially laser radiation, can be introduced into the heart in order to treat tissue.

## Revendications

1. Système pour l'exécution d'interventions diagnostiques et/ou thérapeutiques sur le coeur du type comportant un premier cathéter (1) et un second cathéter (1'), le premier cathéter se composant d'un cathéter guidé (2) d'un cathéter de guidage (3) enveloppant le cathéter guidé (2) et d'un fil de guidage (4) s'étendant dans le lumen (22) du cathéter guidé (2), des moyens étant prévus pour permettre une modification pilotable de l'emplacement du fil de guidage (4) dans un espace partiel du coeur et dans lequel le cathéter est un premier cathéter (1) dont le fil de guidage est réuni ou peut être réuni à l'extrémité distale (30') du second cathéter (1'), le second cathéter pouvant être positionné dans le coeur d'une manière telle que son extrémité distale occupe une position stationnaire indépendante de la modification de l'emplacement du fil de guidage, le second cathéter (1') pouvant être introduit dans le coeur indépendamment du premier cathéter (1) et le second cathéter (1') se composant d'un second cathéter guidé (2'), le second cathéter (1') comportant un organe d'accès avec lequel le fil de guidage (4) du premier cathéter (1) peut être saisi ou peut être tiré dans le second cathéter de manière telle que le premier cathéter (1') et le second cathéter (2') présentent un fil de guidage commun (4).

2. Système selon la revendication 1, **caractérisé en ce que**, pour le fil de guidage commun (4), sont prévues des poignées (7) qui, après introduction du fil de guidage peuvent être saisies aux deux extrémités de celui-ci et qui facilitent la manipulation en vue de la modification de l'emplacement du fil.

3. Système selon la revendication 1 ou 2, **caractérisé en ce que** les modes d'amenée des cathéters (1, 1') dans le coeur sont prévus de manière telle que le premier cathéter pénètre dans le coeur par un premier vaisseau sanguin, en particulier la veine sous-clavière et le second cathéter pénètre par un second vaisseau sanguin en particulier la veine fémorale.

4. Système selon l'une des revendications 1 ou 2, **caractérisé en ce que** les modes d'amenée des cathéters (1, 1') dans le coeur sont prévus de manière telle qu'ils pénètrent dans le coeur par le même vaisseau sanguin, en particulier par la veine sous-clavière.

5. Système selon l'une quelconque des revendications 1 à 4,**caractérisé en ce que** l'extrémité distale (30') du second cathéter (1') présente une collerette de butée souple (8) qui est prévue pour la fixation du cathéter dans le coeur et qui peut être glissée vers l'extérieur.

6. Système selon l'une quelconque des revendications 1 à 4, **caractérisé en ce que** l'extrémité distale (30') du second cathéter (1') présente une pointe (9) qui est prévue pour la fixation du cathéter dans le coeur et qui peut en être extraite.

7. Système selon l'une quelconque des revendications 1 à 6, **caractérisé en ce que** le ou les cathéters guidés (2, 2') consistent en des cathéters à électrodes dont les pôles (21, 22, 21', 22') peuvent analyser les potentiels cardiaques et/ou délivrer un courant à haute fréquence en vue de l'ablation de tissu, des palpeurs de température étant en particulier montés aux deux pôles en vue de l'ablation, et qui sont prévus pour une régulation du courant à haute fréquence en vue d'éviter une surchauffe du tissu à traiter.

8. Système selon l'une quelconque des revendications 1 à 7, **caractérisé en ce que** le ou les cathéters guidés (2, 2') contiennent des fibres conduisant la lumière avec lesquelles sont possibles un diagnostic visuel ou un contrôle des étapes d'un traitement et/ou avec lesquelles peut être introduite une énergie lumineuse en particulier une irradiation par laser en vue du traitement de tissus dans le coeur.
